# EUROPEAN PATENT SPECIFICATION

(11) **EP 1 618 875 B1**
(45) Date of publication and mention of the grant of the patent: **10.09.2008**
(21) Application number: 04726643.2
(22) Date of filing: 08.04.2004
(51) Int. Cl.: A61K 31/12, A61P 19/10, A23L 1/30, A23L 2/38

(54) **COMPOSITION FOR INHIBITION OR PREVENTION OF BONE DENSITY LOWERING**
ZUSAMMENSETZUNG ZUR HEMMUNG ODER PRÄVENTION DER VERRINGERUNG DER KNOCHENDICHTE
COMPOSITION DESTINEE A L'INHIBITION OU A LA PREVENTION DE LA BAISSE DE LA DENSITE OSSEUSE

(30) Priority: 08.04.2003 JP 2003104515
(43) Date of publication of application: 25.01.2006
(73) Proprietor: KIRIN BEER KABUSHIKI KAISHA, Tokyo 104-8288 (JP)
(72) Inventor: TAJIMA, Osamu, c/o Research Center for Product Safety and Assesment, Takasaki-Shi, Gunma-Ken (JP); KATAYAMA, Mikio c/o Research Center for Product Safety and Assesment, Takasaki-Shi, Gunma-Ken (JP)
(74) Representative: HOFFMANN EITLE
(86) International application number: PCT/JP2004/005087
(87) International publication number: WO 2004/089359

(56) References cited:
- EP-A- 1 277 473
- EP-A- 1 283 037
- WO-A-01/03687
- WO-A-02/085393
- WO-A-20/04087181
- WO-A1-03/014287
- JP-A- 7 285 856
- JP-A- 7 330 594
- STEVENS J F ET AL: "Quantitative analysis of xanthohumol and related prenylflavonoids in hops and beer by liquid chromatography-tandem mass spectrometry" JOURNAL OF CHROMATOGRAPHY A, ELSEVIER, AMSTERDAM, NL, vol. 832, no. 1-2, 5 January 1999 (1999-01-05), pages 97-107, XP004156169 ISSN: 0021-9673
- TATSUTSUMI O. ET AL.: 'Kotsusoshosho model rat ni okeru beer ni fukumareru hop yurai seibun no honemitsudo gensho yokusei koka' DAI 57 KAI THE JAPANESE SOCIETY OF NUTRITION AND FOOD SCIENCE TAIKAI KOEN YOSHISHU 01 April 2003, page 203, XP002984703
- STEVENS J.F. ET AL.: 'Fate of xanthohumol and related prenylflavonoids from hops to beer' JOURNAL OF AGRICULTURAL AND FOOD CHEMISTRY vol. 47, no. 6, 1999, pages 2421 - 2428, XP002980501

## Description

### [BACKGROUND OF THE INVENTION]

### Field of the Invention

The present invention relates to a composition and food and drink for the inhibition or prevention of a bone density reduction. More particularly, the present invention relates to a composition for the prevention, treatment or amelioration of osteoporosis, and food and drink for the prevention, treatment or amelioration of osteoporosis.

### Background Art

Osteoporosis is a disease characterized by low bone mass and a failure of the fine structure of osseous tissue and leads to an increase in brittleness of bone and an increase in the risk of fracture. Osteoporosis is becoming a worldwide serious health issue, and one out of three of women in menopause and most of aged persons (both women and men) suffer from osteoporosis. For example, in Japan, osteoporosis-derived fracture is a ternary cause of "bedridden persons," and one of causes which lead to swelling of medical expenses. Further, it is considered that the advance of aging society leads to a further increased number of osteoporosis patients. For this reason, the development of a medicament for preventing, treating or ameliorating osteoporosis can be said to be very important.

It is considered that a factor of a bone mass reduction which gradually progresses after 40 years old is a reduction in calcium absorption from the intestinal tract due to aging or an increase in secretion of parathyroid hormone for keeping homeostasis regarding calcium due to a lowering in the amount of the internal vitamin D₃. Among others, for women, in many cases, female hormones, which function to promote osteogenesis and inhibit bone resorption, are reduced in menopause, resulting in a rapid bone mass reduction.

Up to now, therapeutic agents for osteoporosis which have been used clinically include, for example, vitamin D preparations, calcitonin preparations, estrogen preparations, ipriflavone preparations, vitamin K₂ preparations, protein anabolic steroid preparations, and bisphosphonate preparations. Among them, estrogen preparations are known to be effective for the prevention or treatment of postmenopausal osteoporosis, but on the other hand, they are known to cause side effects such as metrorrhagia, and an increase in a risk of mammary cancer or uterine cancer.

The medicinal treatment of osteoporosis should be in many cases continued for a long period of time, and, thus, various problems such as development of side effects caused by administration of the medicament over a long period of time are not negligible. For this reason, up to now, the medicinal treatment of osteoporosis does not aim at fundamental treatment but aims at symptomatic treatment, for example, for inhibiting the progress of the disease.

Further, regarding osteoporosis, taking preventative measure has been considered to be very important. However, daily prevention of osteoporosis by the administration of a medicament could not have been realized for cost effectiveness and safety reasons.

Accordingly, prophylactic or therapeutic agents for osteoporosis, which are highly safe and daily usable, have been desired, and finding prophylactic or therapeutic agents for osteoporosis from edible foods is favorable from the viewpoint of safety.

On the other hand, in order to prevent osteoporosis through a method other than the medicament administration therapy, proper intake of various nutrients including calcium and, at the same time, an improvement in living habit such as restriction of excessive alcohol intake, quitting of smoking, and exercise have been recommended. Since medicaments are a foreign matter for the living body and treatment with side effect being kept in mind is necessary, nonmedicinal therapy is effective. Among others, treatment through daily diets is considered to be important, and, thus, the development of excellent food products which are effective for the preventing, treatment or amelioration of osteoporosis has been desired.

Hop plants are herbaceous perennials belonging to the family Moraceae (nomenclature: Humulus luplus) native to Europe, and, in general, the cone thereof (matured female flower) is called a hop. The hop is famous as a bitterness and flavoring ingredient for beer and has been ingested by people for years. The bitterness and flavoring of the beer are originated from a lupulin part of the hop (yellow granules formed on the root of internal bract in the cone). The hop is also used as a folk medicine and is known to have physiological effects such as tranquilizing effect, falling sleep and quiet sleep effects, appetite stimulation, stomachic action, and diuretic action.

Humulons and xanthohumol as hop components have been proven to have bone resorption inhibitory effect in an *in vitro* experiment (Japanese Patent Laid-Open Publication Nos. 330594/1995 and 285856/1995, and Biosci Biotech Biochem 61 (1), 158-159 (1997)). It has also been reported that the intake of beer can inhibit a bone mineral density reduction and this activity is derived from the hop (Mikio Katayama et al., "Kotsu Soshosho Moderu Ratto Niokeru Biru No Kotsu Mitsudo Gensho Yokusei Koka (Inhibitory effect of beer against bone mineral density reduction in osteoporosis model rats)"), Proceedings of the 56th Conference and Scientific Meeting of The Japanese Society of Nutrition and Food Science (The Japanese Society of Nutrition and Food Science), issued June 20, 2002, 326 p, 31-10 p).

It has hitherto been known that the hop has estrogen activity (for example, Munch. Med. Wschr. 95, 845 (1953)). In 1999, 8-prenylnaringenin has been isolated as a female hormone active substance from hops (J. Clinical Endocrinology & Metabolism 83 (6), 2249-2252 (1999)). Accordingly, based on this activity, the utilization of a hop concentrate having a significantly enhanced 8-prenylnaringenin content in the prevention or treatment of postmenopausal osteoporosis is considered effective. In this case, however, as with the estrogen preparations, there is a fear of causing side effects.

Isomerization products of the hop component include, for example, isohumulons (iso-α acids) and isoxanthohumol. These are produced in beer by isomerization of the added hop component in the course of beer production (in the step of wort boiling). Incidentally, there is a report that the content of isoxanthohumol in beer is 0.04 to 3.4 mg/L (J. Chromatogr. A 832 (1999) p 97-107). Likewise, the content of isohumulons in beer is about 60 mg/L for a possibly highest-isohumulons content-type beer (Foreign Extra Stout: manufactured by Guinness Malaysisa Berhad: source Brauindustrie).

WO 01/76614 discloses food products, beverages and the like containing a plant-derived growth factor production enhancing substance, and isoxanthohumol is mentioned as the growth factor production enhancing substance. This publication further discloses that this growth factor production enhancing substance exhibit hepatocyte growth factor (HGF) and nerve growth factor (NGF) production enhancing action and can be utilized in treatment of diseases such as hepatopathy, gastrointestinal injury, pneumonia and diabetes.

However, the osteoporosis amelioration activity of isoxanthohumol has never been known so far as the present inventors know. Japanese Patent Laid-Open Publication No. 330594/1995 describes isohumulons together with humulons as an active ingredient. In this publication, however, there is no specific evidence about the inhibitory activity of isohumulons against bone resorption.

WO 02/085393 and WO 03/014287 describe the combined use of isoxanthohumol, xanthohumol and 8-prenylnaringenin for treating diseases such as osteoporosis. These references emphasize that the desired effect can only be achieved, if these compounds are used in combination.

### [SUMMARY OF THE INVENTION]

The present inventors have now found that an isomerized hop-derived component contained in beer or the like is highly effective in inhibiting or preventing a bone mineral density reduction. This effect is considered useful for the prevention, treatment or amelioration of osteoporosis. Further, the present inventors have found that this effect is based on isoxanthohumol, and the effect of isoxanthohumol is better than the effect of xanthohumol, an analogue of isoxanthohumol. The present invention has been made based on these finding.

Accordingly, an object of the present invention is to provide a composition and food and drink for use in the prevention, treatment or amelioration of osteoporosis, which are highly safe and active.

The present invention is directed to the use of isoxanthohumol for the manufacture of a composition for the inhibition or prevention of a bone mineral density reduction. The composition for the inhibition or prevention of a bone mineral density reduction according to the present invention may comprise an isoxanthohumol-containing isomerized hop extract as an active ingredient.

Preferred embodiments of the invention become evident from the following description and from the attached dependent claims.

The active ingredient in the composition or food and drink according to the present invention is highly effective in inhibiting or preventing a bone mineral density reduction and, further, is also expected to have the effect of increasing the bone mineral density. This active ingredient is contained in beer and the like that have been drunk as a beverage in the food and drink for many years. Thus, the composition or food and drink according to the present invention, even when a patient or a person who wishes to prevent osteoporosis takes or eats for a long period of time, exhibits substantially no side effect and thus is highly safe. Further, it should be noted that, even when the composition or food and drink according to the present invention is used at a dose of at least 1000 times larger than the dose at which the contemplated effect is attained, any estrogen action against immature rats is not observed. Therefore, according to the present invention, excellent effects regarding the prevention, treatment or amelioration of osteoporosis can be attained while avoiding side effects through estrogen action as in the case of estrogen preparations, for example, while avoiding an increase in a risk of the occurrence of metrorrhagia, breast cancer or uterine cancer.

### [BRIEF DESCRIPTION OF THE DRAWINGS]

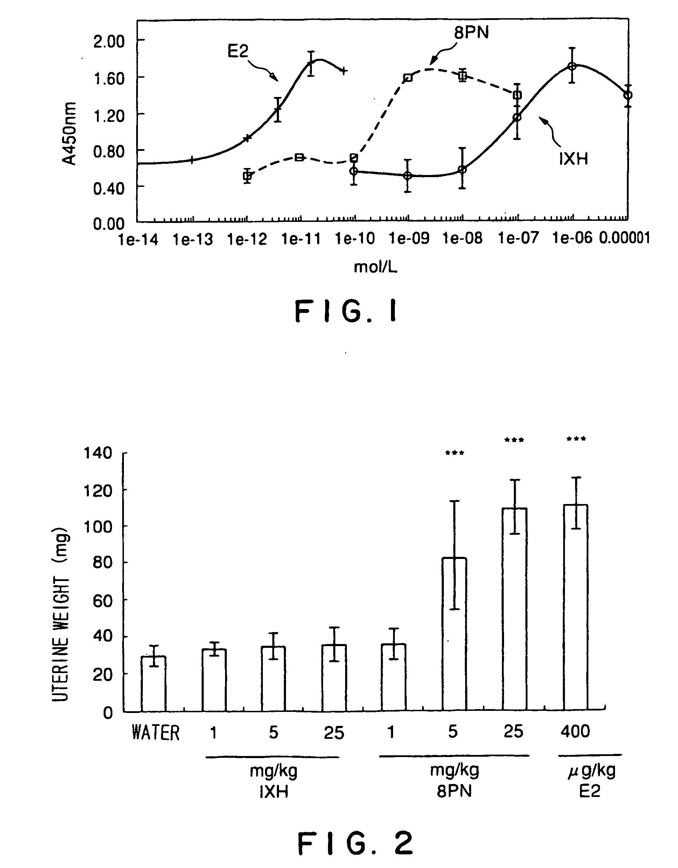

Fig. 1 is a graph showing the results of test 2 in the working example, that is, a graph showing estrogen activity of isoxanthohumol and 8-prenylnaringenin using the growth of MCF-7 cells as an index, wherein IXH represents isoxanthohumol, 8PN represents 8-prenylnaringenin, and E2 represents 17β-estradiol;
Fig. 2 is a graph showing the results of test 3-a in the working example, that is, a graph showing *in vivo* estrogen activity of isoxanthohumol and 8-prenylnaringenin for immature rats, wherein IXH represents isoxanthohumol, 8PN represents 8-prenylnaringenin, and E2 represents 17β-estradiol, and * means P < 0.05 and *** means P < 0.001.

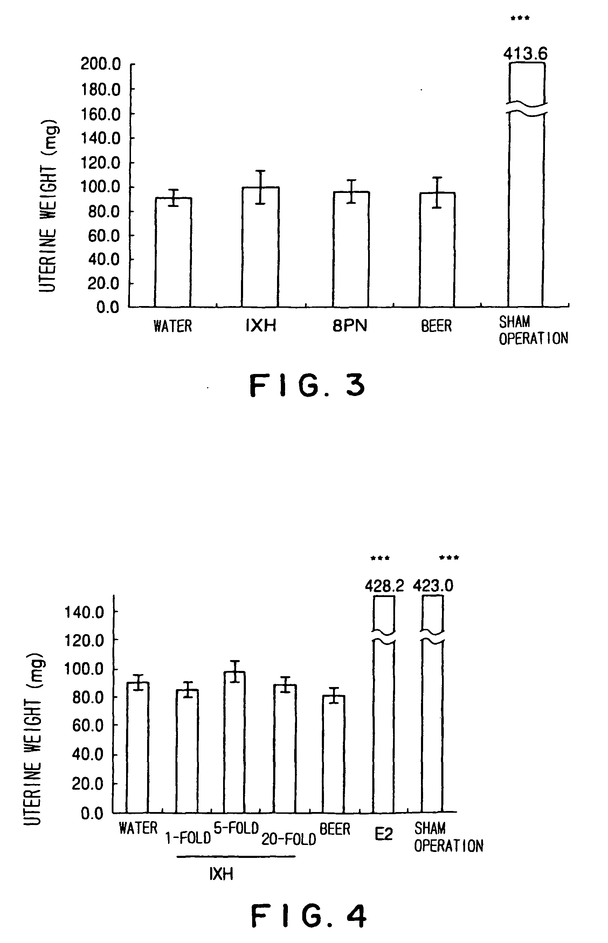

Fig. 3 is a graph showing the results of test 3-b in the working example, that is, a graph showing *in vivo* estrogen activity of isoxanthohumol, 8-prenylnaringenin, and beer for ovariectomized rats, wherein IXH represents isoxanthohumol and 8PN represents 8-prenylnaringenin, and *** means P < 0.001;
Fig. 4 is a graph showing the results of test 3-b in the working example, that is, a graph showing *in vivo* estrogen activity of isoxanthohumol and beer for ovariectomized rats, wherein IXH represents isoxanthohumol and E2 represents 17β-estradiol, and *** means P < 0.001;

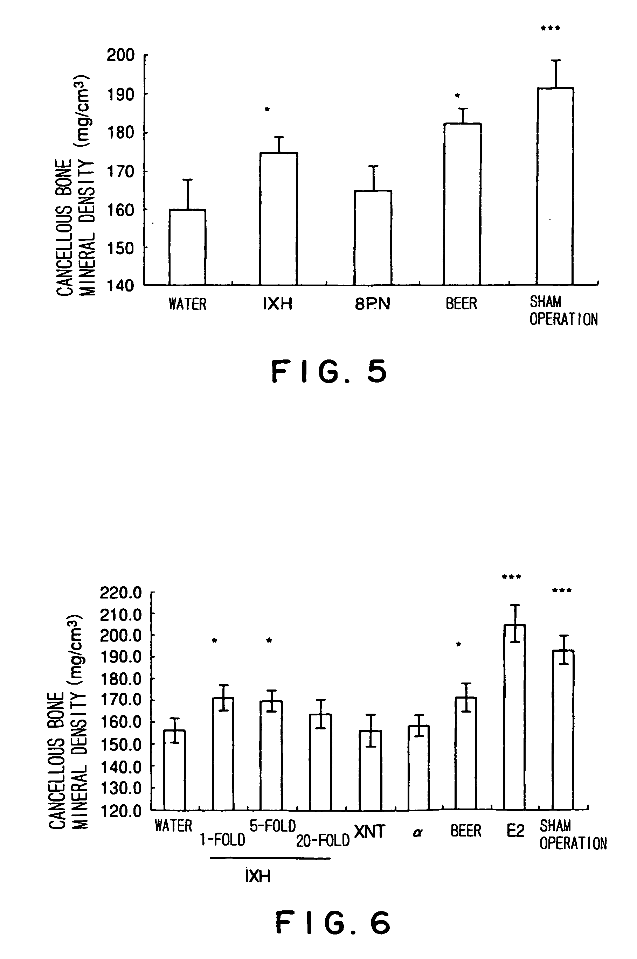

Fig. 5 is a graph showing the results of test 4 in the working example, that is, a graph showing bone mineral density reduction inhibitory activity of isoxanthohumol, 8-prenylnaringenin, and beer for ovariectomized rats, wherein IXH represents isoxanthohumol and 8PN represents 8-prenylnaringenin, and * means P < 0.05 and *** means P < 0.001;
Fig. 6 is a graph showing the results of test 4 in the working example, that is, a graph showing bone mineral density reduction inhibitory activity of isoxanthohumol, xanthohumol, iso-α acid, and beer for ovariectomized rats, wherein IXH represents isoxanthohumol, XNT represents xanthohumol, α represents an iso-α acid-containing hop extract, and E2 represents 17β-estradiol, and * means P < 0.05 and *** means P < 0.001;

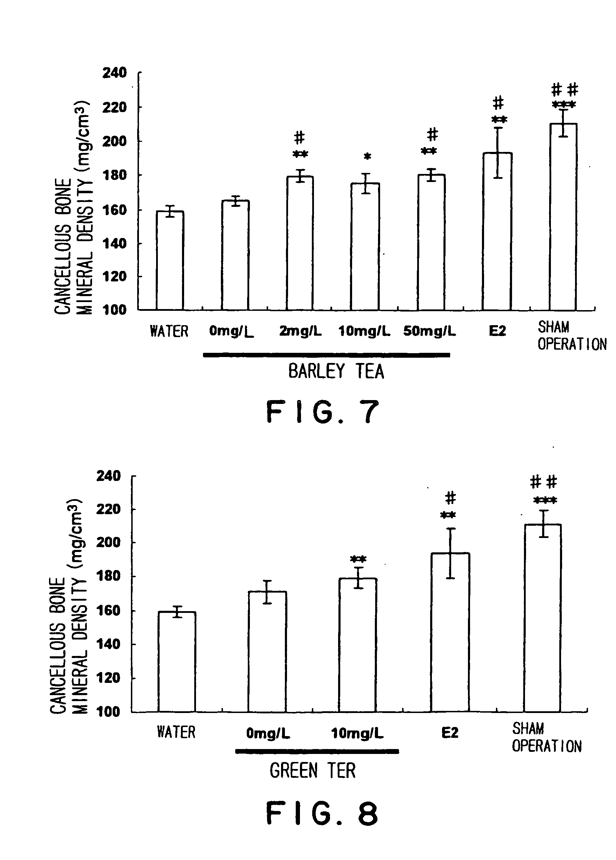

Fig. 7 is a graph showing the results of test 5 in the working example, that is, a graph showing bone mineral density reduction inhibitory activity of barley tea with the addition of isoxanthohumol for ovariectomized rats, wherein 0 mg/L represents barley tea without the addition of isoxanthohumol, 2 mg/L, 10 mg/L, and 50 mg/L represent respective isoxanthohumol concentrations for barely teas with the addition of isoxanthohumol, and E2 represents 17β-estradiol, and, for a control group with the administration of water, * means p < 0.05, ** means p < 0.01, and *** means P < 0.001, while, for barley tea without the addition of isoxanthohumol, # means p < 0.05, and ## means p < 0.01; and
Fig. 8 is a graph showing the results of test 5 in the working example, that is, a graph showing bone mineral density reduction inhibitory activity of green tea with the addition of isoxanthohumol for ovariectomized rats, wherein 0 mg/L represents green tea without the addition of isoxanthohumol, 10 mg/L represents green tea with the addition of isoxanthohumol at an isoxanthohumol concentration of 10 mg/L, and E2 represents 17β-estradiol, and, for a control group with the administration of water, * means p < 0.05, ** means p < 0.01, and *** means p < 0.001, while, for green tea without the addition of isoxanthohumol, # means p < 0.05, and ## means p < 0.01.

### [DETAILED DESCRIPTION OF THE INVENTION]

### Isoxanthohumol

As described above, the composition and food and drink according to the present invention comprises, as an active ingredient, isoxanthohumol or an isoxanthohumol-containing isomerized hop extract.

Isoxanthohumol may be produced by isomerizing commercially available xanthohumol (for example, available from Apin Chemical) or high-purity xanthohumol purified from a hop.

Xanthohumol may be purified from a hop, for example, by the following method. A dried hop is extracted with ethanol, the extract is filtered, and the filtrate is then concentrated under the reduced pressure. Subsequently, the concentrate is subjected to liquid-liquid distribution with hexane and a 85% aqueous ethanol solution to separate an aqueous ethanol solution phase which is then concentrated under the reduced pressure. The concentrate is dissolved in chloroform : hexane (5 : 2), the solution is passed through a silica gel column, and a xanthohumol eluted fraction is separated from among the obtained fractions. The eluate of the separated fraction is concentrated under the reduced pressure, and recrystallization of hexane and ethyl acetate is repeated to give purified xanthohumol.

Xanthohumol may be isomerized by heating xanthohumol in the presence of an alkali or magnesium oxide or by subjecting xanthohumol to other conventional method.

A specific example of isomerization will be described. Xanthohumol is dissolved in an alcoholic solvent such as ethanol. Weakly alkaline water is added to the solution. The mixture is heated under reflux (for example, about 92 to 93°C) in the presence of the weakly alkaline water to thermally isomerize xanthohumol and thus to give isoxanthohumol. The isoxanthohumol produced by the isomerization may be if necessary concentrated or purified by a conventional method, for example, filtration, concentration under the reduced pressure, or lyophilization.

### Isomerized hop extract

In the present invention, the isomerized hop extract refers to a hop extract produced by isomerizing a xanthohumol-containing hop extract obtained, for example, by extraction from a hop. Specifically, the isomerized hop extract is basically produced by providing an extract (a hop extract) derived from a lupulin part in the hop and positively isomerizing the extract.

The hop extract can be prepared, for example, by subjecting hop cone or its compressed product as such or after grinding to extraction operation.

An example of extraction operation is an extraction method using an ethanol solvent which is used as a hop extract preparation method in beer brewing. Alternatively, other commonly used hop extraction methods may be adopted for the extraction operation. Examples of such methods include (1) a method in which a hop cone, its ground product or the like is immersed in a solvent, for example, by maceration or digestion; (2) a method in which extraction is carried out while heating and stirring and extract is obtained by filtration; or (3) percolation.

The crude extract obtained by the above extraction operation may be if necessary subjected to filtration or centrifugation to remove solid matter. The liquid thus obtained as such may be used as a hop extract. Alternatively, prior to use as the hop extract, the solvent contained in the liquid may be removed by evaporation to partially concentrate or dry the liquid. The extract after the concentration or drying may be further washed with an insoluble solvent for purification. This may be further dissolved or suspended in a suitable solvent. Further, in the present invention, before use, the hop extract (liquid) may be treated by conventional means such as drying under the reduced pressure or lyophilization to give a dried product of the hop extract.

Solvents usable in the extraction include: water; and conventional organic solvents, for example, lower alcohols having 1 to 4 carbon atoms such as methanol, ethanol, propanol and butanol; lower alkyl esters such as ethyl acetate; glycols such as ethylene glycol, butylene glycol, propylene glycol, and glycerin; polar solvents such as ethyl ether, acetone and acetic acid; hydrocarbons such as benzene and hexane; and nonpolar solvents, for example, ethers such as ethyl ether and petroleum ether. These solvents may also be used in a combination of two or more.

In one preferred embodiment of the present invention, a hop extract having a higher xanthohumol content can be obtained by providing a xanthohumol-containing extract using a solvent such as ethanol or water and subjecting the extract to supercritical carbon dioxide extraction to remove α acid and β acid fractions contained therein. In this case, if necessary, insolubles may be removed from the hop extract by filtration, or alternatively the hop extraction is evaporated to dryness by concentrating the extract under the reduced pressure or the like to remove the solvent.

The hop extract thus obtained contains at least xanthohumol. The hop extract sometimes contains a very small amount of the isomerization product isoxanthohumol in addition to xanthohumol. In such a case, in the present invention, this hop extract (unisomerizied hop extract) as such may be used. In general, however, this hop extract is further isomerized to give an isomerized hop extract which is then used in the present invention.

The hop extract may be isomerized by heating the hop extract in the presence of an alkali or magnesium oxide, or by subjecting the hop extract to other conventional methods. Thus, xanthohumol contained in the hop extract is converted to isoxanthohumol.

A specific example of isomerization will be described. A xanthohumol-conaining hop extract is dissolved in an alcoholic solvent such as ethanol. Weakly alkaline water is added to the solution. The mixture is heated under reflux (for example, about 92 to 93°C) in the presence of the weakly alkaline water to thermally isomerize the hop extract and thus to give an isoxanthohumol-containing isomerized hop extract. The isomerized hop extract may be if necessary concentrated or purified by a conventional method, for example, filtration, concentration under the reduced pressure, or lyophilization. Commercially available water such as drinkable ionized alkaline water, for example, bottled water is preferred as the weakly alkaline (for example, pH 8.5 to 9.5) water used in the isomerization treatment from the viewpoint of safety. Commercially available drinkable water is highly safe because of satisfactory ingestion experience. Further, since the reaction type in which thermal isomerization takes place in the process of wort boiling in beer brewing is essentially identical to the above isomerization, the safety from the viewpoint of providing food and drink is advantageously high.

Accordingly, in a preferred embodiment of the present invention, the isomerized hop extract is produced by isomerizing a xanthohumol-containing hop extract obtained by extraction with an ethanol solvent. In this case, more preferably, the isomerization is carried out by heating the hop extract in alkaline water under reflux. Here the ethanol solvent refers to, for example, a solvent mainly composed of ethanol or a solvent composed of ethanol and water.

In the present invention, the isomerized hop extract obtained by the isomerization treatment may be used directly in the production of a composition or food and drink. Preferably, however, a fractionation product containing the active ingredient in a higher concentration is obtained and is used.

Accordingly, in a preferred embodiment of the present invention, the isomerized hop extract used as an active ingredient of the composition or food and drink in the present invention may have a higher isoxanthohumol content than that obtained by mere isomerization of the hop extract.

In the present invention, commercially available hop extracts extracted by various methods may be obtained and isomerized to give an isomerized hop extract before use. Alternatively, commercially available isomerized hop extracts as such may be used.

The isomerized hop extract containing isoxanthohumol in a high concentration can easily be produced by providing a conventional isomerized hop extract obtained by isomerizing a hop extract extracted from a hop and then treating the isomerized hop extract by conventional methods, for example, by concentration, addition of isoxanthohumol, or removal of the components other than the isoxanthohumol.

Examples of commercially available hop extracts include a hop extract produced by extracting a hop cone ground product with ethanol and then fractionating the extract by supercritical carbon dioxide extraction to enhance the xanthohumol content (for example, Xanthohumol-enriched hop product (tradename), available from Hopsteiner) and a product containing about 80% of xanthohumol (Xantho-pure (tradename), available from Hopsteiner). They can be isomerized to give an isomerized hop extract having a high isoxanthohumol content.

It is needless to say that a fractionation product having a higher active ingredient content can be produced from these extracts by concentration by methods including the above method.

In a more preferred embodiment of the present invention, the isomerized hop extract used as an active ingredient of the composition or food and drink according to the present invention comprises 0.005 to 90% by weight, more preferably 8 to 90% by weight, of isoxanthohumol.

Further, in the food and drink, when the production process of food and drink includes the step of isomerizing xanthohumol, such as the step of heating, the above hop extract, preferably a hop extract having a xanthohumol content, which has been rendered higher than that of the conventional hop extract, may be added before the step of heating the food and drink so that the final product contains isoxanthohumol. Representative examples of such foods and drinks include beer and low-malt beer. In the beer and low-malt beer, the above hop extract may be added in the step of wort boiling so that the final product contains isoxanthohumol. When the xanthohumol-enriched hop extract is used in the above manner, products having a high isoxanthohumol content can be produced without enhancing the concentration of isohumulons as a bitterness component in beer to a level more than necessary (that is, without imparting an excessive level of bitterness). In this connection, it should be noted that the presence of this step of isomerization does not exclude the above-described embodiment in which purified isoxanthohumol, an isomerized hop extract having a high isoxanthohumol content obtained by isomerizing, for example, a hop extract having an enhanced xanthohumol content (for example, Xanthohumol-enriched hop product (tradename), available from Hopsteiner), or a product having a xanthohumol content of about 80% (Xantho-pure (tradename), available from Hopsteiner) is added in the production process or to the final product.

### Use

In ovariectomized animals and humans, osteoclast activation and bone resorption enhancement are observed. In general, however, when a female hormone is added, a bone mass reduction phenomenon caused by the osteoclast activation and bone resorption enhancement disappears. How this action is developed has not been elucidated yet. However, it is considered that, for example, cytokine IL1, cytokine IL6, TGFβ, prostaglandin E and the like involved in bone resorption participate in this action in a complicated manner. In addition to an enhancement in bone resorption by a shortage of a female hormone, suppression of calcium absorption from the intestinal tract is also considered as a cause of a bone mass reduction.

Upon combining with a nuclear receptor, the female hormone develops the activity. In recent years, it has become apparent that two types of receptors, α and β, exist. Further, in recent years, the development of medicaments (SERMs), which act as a female hormone on a certain organ but act antagonistically on another tissue, progresses. These medicaments are expected as medicaments having no significant side effect. An attempt has been made to explain, through two receptor types, the mechanism in which the action of the medicament on a certain organ is different from the action on another organ.

There are complicated and a wide variety of onset mechanisms for post menopausal osteoporosis, and a large part thereof has not been elucidated yet. Therefore, the effectiveness of prevention or treatment of postmenopausal osteoporosis cannot be satisfactorily explained only from the estrogen agonist activity. In fact, for example, in Japan, due to side effect, preparations having no estrogen agonist activity are mainly used clinically.

As shown in test 4 in the working example using ovariectomized rats which will be described later, isoxanthohumol significantly improved a bone mineral density reduction caused by blocking of estrogen secretion from the ovary. Based on this fact, it is estimated that isoxanthohumol acts directly or indirectly on osteoclasts to suppress bone resorption. On the other hand, test 2 in the working example showed that isoxanthohumol has estrogen activity in an *in vitro* experiment. In this case, in immature rats, even at a dose of at least 1000 times larger than the dose at which bone mineral density reduction amelioration activity was developed in ovariectomized rats, uterine hypertrophy action was not observed (see test 3 in the working example which will be described later). Based on this, it is estimated that isoxanthohumol has a higher selectivity for osseous tissue than for uterine, although it has a mechanism different from 17β-estradiol, a female hormone, that is, has interaction with an estrogen receptor.

Accordingly, pharmacotherapy using conventional estrogen preparations has a fear of causing side effects such as metrorrhagia, and an increase in a risk of occurrence of breast cancer and uterine cancer, whereas the active ingredient according to the present invention can prevent, treat, or ameliorate osteoporosis while avoiding the above side effects.

Accordingly, the composition or food and drink according to the present invention can be used for the inhibition, amelioration or prevention of a bone mineral density reduction. If possible, the composition or food and drink according to the present invention may be used for increasing the bone mineral density. Therefore, the composition or food and drink according to the present invention can be used for the prevention, treatment or amelioration of osteoporosis.

Here the term "bone mineral density" refers to the amount of minerals such as calcium per a given volume in bone of an object mammal, and the bone mineral density can be measured by a conventional method. Examples of the conventional method include methods using X rays (for example, DXA, MD, and pQCT methods) and methods using ultrasonic waves (for example, QUS method). In the present invention, the bone mineral density may be measured, for example, by the method described in test 4 in the working example which will be described later.

According to the present invention, there is provided a method for the inhibition or prevention of a bone mineral density reduction of mammals, said method comprising the step of administering an effective amount of isoxanthohumol or an isoxanthohumol-containing isomerized hop extract to a mammal or allowing a mammal to ingest an effective amount of isoxanthohumol or an isoxanthohumol-containing isomerized hop extract. Further, there is provided a method for the prevention, treatment, or amelioration of osteoporosis, said method comprising the step of administering a prophylactically, therapeutically or amelioratively effective amount of isoxanthohumol or an isomerized hop extract to a mammal or allowing a mammal to ingest a prophylactically, therapeutically or amelioratively effective amount of isoxanthohumol or an isomerized hop extract. Here the "effective amount" can be selected according to the dose of isoxanthohumol which will be described later.

According to another aspect of the present invention, there is provided use of isoxanthohumol or an isomerized hop extract, for the manufacture of a composition for the inhibition or prevention of a bone mineral density reduction. Further, according to a further aspect of the present invention, there is provided use of isoxanthohumol or an isomerized hop extract, for the manufacture of a composition for the prevention, treatment, or amelioration of osteoporosis.

### Composition and food and drink

The composition according to the present invention comprises isoxanthohumol or an isomerized hop extract as an active ingredient.

Here "comprising as an active ingredient" means that a pharmaceutically acceptable carrier according to the desired formulation may of course be incorporated and, in addition, other medicaments usable in combination with the composition of the present invention may be incorporated.

The composition according to the present invention may be administered orally or parenterally. Specifically, oral preparations include granules, powders, tablets (including sugar coated tablets), pills, capsules, syrups, emulsions, and suspensions. Parenteral preparations include injections (for example, subcutaneous injections, intravenous injections, intramuscular injections and intraperitoneal injections), drops, external preparations (for example, transnasal preparations, transdermal preparations, and ointments), and suppositories (for example, rectal suppositories and pessaries). These preparations may be prepared by a method commonly used in the art with a pharmaceutically acceptable carrier.

Pharmaceutically acceptable carriers include excipients, binders, diluents, additives, perfume, buffer agents, thickening agents, coloring agents, stabilizers, emulsifiers, dispersants, suspending agents, and preservatives. For example, magnesium carbonate, magnesium stearate, talc, sugar, lactose, pectin, dextrin, starch, gelatin, tragacanth, methylcellulose, sodium carboxymethylcellulose, low-melting wax, and cacao butter may be used as the carrier.

Other medicaments usable in combination with the active ingredient according to the present invention include, for example, amino acid, antibiotics, vitamin D preparations, calcitonin preparations, estrogen preparations, ipriflavone preparations, vitamin K₂ preparations, protein anabolic steroid preparations, and bisphosphonate preparations.

Preparations may be manufactured, for example, by the following methods.

Oral preparations may be manufactured, for example, by adding excipients (for example, lactose, saccharose, starch and mannitol), disintegrators (for example, calcium carbonate and carboxymethylcellulose calcium), binders (for example, gelatinized starch, gum arabic, carboxymethylcellulose, polyvinyl pyrrolidone, and hydroxypropylcellulose), or lubricants (for example, talc, magnesium stearate, and polyethylene glycol 6000) to the active ingredient, compressing the mixture, and optionally coating the compression product with a coating for taste masking, enteric or persistence purposes by a method known per se. Coating agents usable herein include, for example, ethyl cellulose, hydroxymethylcellulose, polyoxyethylene glycol, cellulose acetate phthalate, hydroxypropylmethylcellulose phthalate, and Eudragit (manufactured by Roehm Pharma, Germany; methacrylic acid-acrylic acid copolymer), gelatin, and pullulan.

Injections may be manufactured, for example, by dissolving, suspending, or emulsifying the active ingredient together with dispersants (for example, Tween 80 (manufactured by Atlas Powder Co.,) (US), HCO 60 (manufactured by Nikko Chemicals Co., Ltd.), polyethylene glycol, carboxymethylcellulose, sodium alginate and the like), preservatives (for example, methylparaben, propylparaben, benzyl alcohol, chlorobutanol, and phenol), tonicity adjusting agents (for example, sodium chloride, glycerin, sorbitol, glucose, and invert sugar) or the like, for example, in aqueous solvents (for example, distilled water, saline, Ringer solution and the like) or oily solvents (for example, vegetable oils such as olive oil, sesame oil, cotton seed oil and corn oil, and propylene glycol). In this case, if necessary, additives such as solubilizers (for example, sodium salicylate and sodium acetate), stabilizers (for example, human serum albumin), and soothing agents (for example, benzalconium chloride and procaine hydrochloride) may be added.

External preparations may be manufactured by bringing the active ingredient to a solid, semi-solid or liquid composition. For example, the solid composition may be prepared by bringing the active ingredient as such to powder, or by adding and mixing excipients (for example, lactose, mannitol, starch, microcrystalline cellulose and saccharose), thickening agents (for example, naturally occurring gums, cellulose derivatives and acrylic acid polymer) or the like with the active ingredient and bringing the mixture to powder. The liquid composition can be manufactured substantially in the same manner as in the injections. The semi-solid composition may be in an aqueous or oily gelling agent or cartilaginous form. Further, any of these compositions may further comprise pH adjustors (for example, carbonic acid, phosphoric acid, citric acid, hydrochloric acid, or sodium hydroxide), preservatives (for example, p-oxybenzoic esters, chlorobutanol, and benzalconium chloride) and the like.

Suppositories may be manufactured by bringing the active ingredient to an oily or aqueous solid, semi-solid or liquid composition. In this case, an oily or aqueous base may be used. Oily bases usable in the composition include glycerides of higher fatty acids (for example, cacao butter, and Wittep sol (manufactured by Dynamit Nobel), middle fatty acids (for example, Miglyol, manufactured by Dynamit Nobel), or vegetable oils (for example, sesame oils, soybean oils, and cotton seed oils). Aqueous bases include polyethylene glycols and propylene glycol. Aqueous gel bases include naturally occurring gums, cellulose derivatives, vinyl polymers, and acrylic acid polymers.

The food and drink according to the present invention comprises an effective amount of the active ingredient according to the present invention.

Here the "comprising an effective amount of the active ingredient" means that the active ingredient is contained in such an amount that, as a result of intake of individual food and drink in such an amount that is usually eaten, the active ingredient is ingested in an amount range which will be described later. The active ingredient according to the present invention as such may be incorporated in the above form of composition into the food and drink according to the present invention. More specifically, foods and drinks according to the present invention may be food and drink prepared by bringing the active ingredient according to the present invention or the above hop ground product or extract as such or after isomerization to food and drink, food and drink prepared by further incorporating various proteins, saccharides, fats, trace elements, vitamins and the like thereinto, or food and drink prepared by adding the food and drink according to the present invention to conventional food and drink.

In the present invention, the "food and drink" is not particularly limited so far as they are other than medicaments and can be ingested by mammals, and the form of the food and drink may be any of liquids, semi-liquids, or solids. Accordingly, the food and drink embrace, for example, a beverage form. Further, the food and drink embraces those belonging to the category of health foods, functional foods, foods for specified health use, and foods for sick persons. Further, the "food and drink," when used for mammals other than humans, may embrace feeds.

The active ingredient in the present invention has inhibitory or prophylactic activity against a bone mass reduction. Accordingly, food and drink which exhibit a combination of a contemplated inherent function with prevention of onset, treatment and amelioration of osteoporosis, and the function of preventing transiting a preliminary group of osteoporosis to osteoporosis can be provided by incorporating the active ingredient according to the present invention into daily ingested foods and health foods and functional foods ingested as supplements, suitably into a food or the like containing one or more minerals such as calcium and magnesium, and vitamins such as vitamin K.

Thus, the food and drink according to the present invention can be provided as food and drink suitable for consumers who wish to enhance bone mineral density or to prevent a lowering in bone mineral density, or food and drink suitable for consumers who worry about the health of bone, that is, the so-called foods for specified health use.

The food for specified health use used herein refer to food and drink which, in the manufacture or sale or the like for bone mineral density reduction inhibition or prevention purposes, undergo a certain limitation in law in various countries from the viewpoint of health.

Specific examples of foods and drinks include carbohydrate-containing foods and drinks such as rice, noodle, bread, and pasta; various confectioneries including western confectioneries such as cookies and cakes, Japanese confectioneries such as steamed bean-jam bun and sweet jelly of beans, candies, gums, frozen desserts such as yogurt and pudding, iced desserts and the like; various beverages including juice, soft drink, milk beverage, tea beverage, functional drink, nutritious supplement drink, alcohol-free beer and the like; alcoholic beverages such as beer and low-malt beer; and processed food using eggs or processed food (including dainties) using seafood (cuttlefish, octopus, shellfish, eel and the like) or animal meat (including variety meat such as liver).

The food and drink are preferably non-grain-hop fermentation-type food and drink. The "non-grain-hop fermentation-type food and drink" used herein refer to food and drink that are free from a fermentation component (for example, beer and low-malt beer) produced by fermentation using at least a hop and grains as the raw material. That is, the non-grain-hop fermentation-type food and drink refer to food and drink other than fermented drinks containing a hop as the raw material such as beer and low-malt beer. The recovery, maintenance, or progress of bone health can be realized by providing non-grain-hop fermentation-type food and drink and adding an effective amount of isoxanthohumol to the food and drink.

In one more preferred embodiment of the present invention, foods and drinks include tea beverages, milk beverages, and yoghurt. In another one preferred embodiment of the present invention, the food and drink is a beverage.

The tea beverage refers to a beverage in which, for example, leaves (tea leaves) of a tea plant (an evergreen tree of the family Theaceae), or leaves of plants other than the tea plant or grains are decocted for drinking. The tea beverages embrace all of fermented tea, semi-fermented tea and unfermented tea. Specific examples of tea beverages include Japanese tea (for example, green tea or barley tea), black tea, herb tea (for example, jasmine tea), Chinese tea (for example, Chinese green tea, oolong tea), or roasted tea.

The milk beverage refers to raw milk, cow milk or the like, or beverages produced using as a main raw material foods produced using them as the raw material. The milk beverage embraces beverages using as the raw material cow milk or the like per se, and, further, beverages using as the raw material, for example, processed milk such as nutrient enriched milk, flavor added milk, and sweetened degraded milk.

Yoghurt includes any of hard type, soft type, and drink type and, further, processed yoghurt products produced using yoghurt as the raw material.

When a product obtained by isomerizing the active ingredient according to the present invention or a ground product or extract of a hop is incorporated into a raw material of conventional food to prepare a food, as will be described later, preferably, the active ingredient or the ground product or extract is used in such an amount that the bitterness of the active ingredient or the hop does not affect the taste of the food and drink, or a technique is adopted in which the bitterness is masked.

In general, hop-derived food and drink such as beer, alcohol-free beer, and low-malt beer sometimes already contain a given or larger amount of the active ingredient according to the present invention. That is, such conventional isoxanthohumol-containing food and drink are not food and drink to which a hop component has been merely added but food and drink produced via the step of isomerizing the hop component. In the case of such food and drink, they as such can be used as the food and drink according to the present invention. However, the incorporation of isoxanthohumol or an isoxanthohumol-containing extract, or an isomerized hop extract into these food and drink can further enhance the desired effect or can reduce the effective intake.

Examples of conventional isoxanthohumol-containing food and drink include beer. As described above, the content of isoxanthohumol in the beer is, for example, 0.04 to 3.4 mg/L. However, isohumulons as a bitterness component and 8-prenylnaringenin having estrogen activity are also contained in the beer. For example, the content of 8-prenylnaringenin in the beer is known to fall within a broad range of 1/6 to 1/133 of the isoxanthohumol content (J. Chromatogr. A 832, 97-107 1999).

In the food and drink according to the present invention, the use of an isomerization product of a hop extract is considered to be advantageous over the use of purified isoxanthohumol from the viewpoint of cost.

When an isomerized hop extract is used in the food and drink, preferably, a sensory aspect derived from bitterness created by isohumulons or the like is also taken into consideration. In this case, preferably, as compared with the content of isoxanthohumol in food and drink, the content of isohumulons in the food and drinks is relatively lowered by using a xanthohumol enriched hop extract or an isoxanthohumol enriched isomerized hop extract. Alternatively, a method may be adopted in which the bitterness component is masked, and, further, the amount of the hop, hop extract, or isomerized hop extract added is increased.

In the food and drink, when there is substantially no problem of cost, purified isoxanthohumol may be added in the course of producing a food or to the final product. Alternatively, when the production process involves a step which brings about an isomerization reaction, purified xanthohumol may be added before this step.

The composition or food and drink according to the present invention use a hop extract component or its derivative which have been ingested as food and drink by humans for many years and thus are low in toxicity. Thus, they can be safely used for mammals (for example, human, mouse, rat, rabbit, dog, cat, cattle, horse, pig, and monkey).

When the composition according to the present invention is used for increasing the bone mineral density of a mammal to which the composition according to the present invention is administered, or for inhibiting, ameliorating, or preventing a bone mineral density reduction of a mammal to which the composition according to the present invention is administered (preferably when the prevention, treatment, or amelioration of osteoporosis is contemplated), the dose may be determined by taking into consideration, for example, recipients, age and body weight of recipients, symptoms, administration period, dosage form, administration method, and a combination of medicaments. For example, when the active component according to the present invention is administered as a medicament, the dose is 0.01 to 100 mg/kg- body weight adult, preferably 0.02 to 50 mg/kg-body weight adult, for oral administration, and 0.005 to 10 mg/kg- body weight, preferably 0.01 to 10 mg/kg- body weight, for parenteral administration. This dose may be administered at a time daily or divided doses of several times daily.

When the isomerized hop extract is used as the active ingredient, it may be administered so that the intake per adult is not more than 5 mg/kg-weight·day (intake: not more than 300 mg per day per adult), preferably not more than 1 mg/kg- body weight·day, in terms of the amount of isoxanthofumol.

In a preferred embodiment of the present invention, the composition according to the present invention comprises isoxanthohumol in such an amount that the intake of isoxanthohumol per day per adult is 0.003 to 5 mg/kg-weight·day (0.2. to 300 mg in terms of intake of isoxanthohumol per day per 60 kg-body weight adult). More preferably, the lower limit of the intake of isoxanthohumol per adult is not less than 0.013 mg/kg-body weight.day (not less than 0.8 mg in terms of intake per day per 60 kg-body weight adult), still more preferably, not less than 0.02 mg/kg-body weight·day (not less than 1.2 mg in terms of intake per day per 60 kg-body weight adult).

In a preferred embodiment of the present invention, the food and drink according to the present invention comprises isoxanthohumol in such an amount that the intake of isoxanthohumol per day per adult is 0.003 to 0.5 mg/kg-body weight·day (0.2 to 30 mg in terms of intake of isoxanthohumol per day per 60 kg-body weight adult). More preferably, the lower limit of the intake of isoxanthohumol per adult is not less than 0.013 mg/kg-body weight·day (not less than 0.8 mg in terms of intake per day per 60 kg-body weight adult), still more preferably, not less than 0.02 mg/kg-body weight-day (not less than 1.2 mg in terms of intake per day per 60 kg-body weight adult).

The lower limit of the intake of isoxanthohumol, i.e., 0.003 mg/kg-body weight adult·day (0.2 mg in terms of intake per day per 60 kg-body weight adult) corresponds to the administration of a test reagent, having an isoxanthohumol concentration of 2 mg/L, which has been confirmed to be effective in experiment 4 and experiment 5 in the working example which will be described later, at a dose of 10 ml/kg. More specifically, this lower limit is a value obtained by conversion of the dose from the ratio between the body surface area of rat and the body surface area of human. The intake per adult obtained by conversion of the dose to a value per body weight of rat is 0.02 mg/kg-body weight·day (1.2 mg of intake per day per 60 kg-body weight adult).

For the food and drink, the upper limit of the intake of isoxanthohumol, that is, 0.5 mg/kg-body weight adult·day, 30 mg in terms of intake per day per 60 kg-body weight adult was determined by taking into consideration the solubility of isoxanthohumol and dietary experiences. Even when the intake of isoxanthohumol is larger than the above upper limit, the bone mineral density reduction inhibitory effect is not negated. Accordingly, if possible, the food and drink may contain isoxanthohumol in an amount exceeding the above upper limit.

In a preferred embodiment of the present invention, the food and drink comprises isoxanthohumol in such an amount that the intake of isoxanthohumol per time is 0.2 to 30 mg, more preferably 0.8 to 30 mg, still more preferably 1.2 to 30 mg.

The expression "intake of isoxanthohumol per time" as used herein refers to the amount of isoxanthohumol contained in the food and drink ingested in eating action per time by a human. Accordingly, in the case of food and drink prepared on the presumption that the product is ingested by single eating action of a human, the "intake of isoxanthohumol per time" corresponds to the amount of isoxanthohumol contained in such food and drink.

The form of the food and drink prepared on the presumption that the product is ingested by single eating action of a human may be properly determined in consideration of physical factors, for example, the weight, age, sex, physical strength, and health condition of an object who is expected to ingest the food and drink, and environment, weather or the like under which the food and drink are expected to be ingested. For example, the form of such food and drink is a beverage filled into a vessel in an amount unit in the range of 100 to 500 ml (preferably 100 to 350 ml).

When the form of the food and drink prepared on the presumption that the product is ingested by single eating action is a 100 ml-bottled beverage, the 100-ml beverage contains isoxanthohumol in an amount corresponding to the above intake of isoxanthohumol (0.2 to 30 mg). Accordingly, the concentration of isoxanthohumol in this beverage is in the range of 2 to 300 mg/L. Likewise, when the form of the food and drink prepared on the presumption that the product is ingested by single eating action is a 350 ml-bottled beverage, the 350-ml beverage contains isoxanthohumol in an amount corresponding to the above intake of isoxanthohumol (0.2 to 30 mg). Accordingly, the concentration of isoxanthohumol in this beverage is in the range of 0.57 to 85.7 mg/L.

Further, a specific example will be described. In the above form of food and drink, the concentrations of isoxanthohumol in bottled beverages (volume unit: 100 ml, 200 ml, and 350 ml) are as shown in Table A below.

**Table A**

| Intake of isoxantho-hum ol per time | Isoxanthohumol concentration, mg/L | | |
|---|---|---|---|
| | 100-ml beverage | 200-ml beverage | 350-ml beverage |
| 0.2 to 30 mg | 2 to 300 | 1 to 150 | 0.57 to 85.7 |
| 1.2 to 30 mg | 12 to 300 | 6 to 150 | 3.43 to 85.7 |

Beverages on the presumption that the desired volume unit is ingested by single eating action can easily be prepared by utilizing of the above table or converting the values in the above table to set the concentration of isoxanthohumol in the beverage.

### [EXAMPLES]

The present invention is further illustrated by the following Examples.

### Production Example:

### Example A: Purification of isoxanthohumol

An example of purification of isoxanthohumol from beer (KIRIN LAGER BEER (tradename), manufactured by Kirin Brewery Co.,LTD. (Japan)) (hereinafter abbreviated to "beer"), and a powdery hop extract having a high xanthohumol content (Xanthohumol Enriched Product (tradename) and Xantho-pure (tradename), both manufactured by Hopsteiner) will be described.

### Purification from beer

Beer (1000 ml) was degassed, and a 800 ml portion thereof was subjected to extraction with 1000 ml of diethyl ether. The diethyl ether phase was dehydrated over anhydrous sodium sulfate and was concentrated under the reduced pressure. Thereafter, 20 ml of ethyl acetate was added, and the mixture was again dehydrated over anhydrous sodium sulfate and was concentrated to about 1 ml under the reduced pressure. The concentrate was fractionated by column chromatography on silica gel (Kieselgel 60, 10 g). The column was washed with 30% ethyl acetate/n-hexane and was then eluted with 60% ethyl acetate/n-hexane. The eluted fraction was evaporated under the reduced pressure to dryness, followed by dissolution in 3 ml of methanol. Further, 3 ml of distilled water was added to the solution, and the mixture was further purified by HPLC. Conditions for HPLC were as follows.
Mobile phase: 45% acetonitrile and 55% distilled water
Column: YMC-ODS-AQ 25 x 250 mm (manufactured by YMC Co., Ltd.)
Guard column: YMC-ODS-AQ 25 x 50 mm (manufactured by YMC Co., Ltd.)
Flow rate: 10 ml/min, injection amount 3 ml
Detector wavelength: UV 290 nm

The purity of the purified isoxanthohumol was determined by HPLC analysis and nuclear magnetic resonance (NMR) spectrum measurement. Conditions for HPLC for purity determination were as follows.
Mobile phase: liquid A (acetonitrile), liquid B (distilled water), and gradient elution with liquid A (20% to 100%)
Column: YMC-hydrosphere C18 4.6 x 250 mm (manufactured by YMC Co., Ltd.)
Flow rate: 1 ml/min, injection amount 5 µl
Detector wavelength: UV 290 nm

As a result of this HPLC analysis, the purity of isoxanthohumol was found to be not less than 98%.

The purified isoxanthohumol (1 mg) was dissolved in 800 ml of methanol-d4, and the solution was analyzed by 1 H-NMR measurement with a nuclear magnetic resonance (NMR) spectrum apparatus (JNM-A400 (manufactured by Japan Electric Optical Laboratory).

As a result of the NMR measurement, it was found that chemical shifts of isolated and purified isoxanthohumol were in agreement with literature values, confirming that the isoxanthohumol had a single composition.

### Purification from hop extract (1)

A hop extract (Xanthohumol Enriched Product (tradename)) (1 g) was added to 1 L of water, and the mixture was boiled in an autoclave at 100°C for 120 min for isomerization. The isomerization product was cooled to room temperature, and the supernatant was then filtered through filter paper to provide a filtrate. Subsequently, 800 ml of the filtrate was extracted with 1000 ml of diethyl ether. The diethyl ether phase was dehydrated over anhydrous sodium sulfate and was concentrated under the reduced pressure. Thereafter, 20 ml of ethyl acetate was added, and the mixture was again dehydrated over anhydrous sodium sulfate and was concentrated to about 1 ml under the reduced pressure. The concentrate was fractionated by column chromatography on silica gel (Kieselgel 60, 10 g). The column was washed with 30% ethyl acetate/n-hexane and was then eluted with 60% ethyl acetate/n-hexane. The eluted fraction was evaporated under the reduced pressure to dryness, followed by dissolution in 3 ml of methanol. Further, 3 ml of distilled water was added to the solution, and the mixture was further purified by HPLC. Conditions for HPLC were as follows.
Mobile phase: 45% acetonitrile and 55% distilled water
Column: YMC-ODS-AQ 25 x 250 mm
Guard column: YMC-ODS-AQ 50 x 25 mm
Flow rate: 10 ml/min, injection amount 3 ml
Detector wavelength: UV 290 nm

The purity of the purified isoxanthohumol was determined by HPLC analysis and nuclear magnetic resonance (NMR) spectrum measurement in the same manner as in the purification from beer.

As a result of HPLC analysis, the purity of isoxanthohumol was found to be not less than 98%.

As a result of the NMR measurements, it was found that chemical shifts of isolated and purified isoxanthohumol were in agreement with literature values, confirming that the isoxanthohumol had a single composition.

### Purification from hop extract (2)

A hop extract (Xantho-pure (tradename)) (2.5 g) was dissolved in 50 ml of ethanol. A 10 N aqueous sodium hydroxide solution (50 ml) and distilled water were added to the solution to bring the volume to 500 ml. The mixture was boiled in a boiling water bath for one hr for isomerization followed by cooling to room temperature. 12 N hydrochloric acid (45 ml) was added thereto. Subsequently, the resultant solution was cooled and was then suction filtered through a glass wool filter. Ether (1.2 L) and 0.8 ml of distilled water were added to the collected product, followed by liquid-liquid distribution extraction twice. The ether phase was evaporated under the reduced pressure to dryness. Minor amounts of ethyl acetate and hexane were added, and recrystallization was repeated to give isoxanthohumol having a purity of not less than 98%.

### Example B: Isomerization of hop extract

A commercially available hop extract having a xanthohumol content of about 80% (Xantho-pure (tradename)) (18 g) was dissolved in 2 L of ethanol. A weakly alkaline (pH 8.5 to 9.5) bottled water (Arukari Ion no Mizu (ionized alkaline water) (tradename), manufactured by KIRIN Beverage) (18 L) was added to the solution. The mixture was heated at 92 to 93°C under reflux for 2 hr for thermal isomerization. Next, insolubles were removed by filtration through filter paper. The filtrate was then concentrated under the reduced pressure, and the concentrate was lyophilized. The lyophilization provided about 18 g of a powdery isomerized hop extract. The powdery isomerized hop extract had an isoxanthohumol content of about 70%.

Purification in the same manner as in the column of "Purification from hop extract (2)" in Example A could provide isoxanthohumol having a purity of not less than 98%.

### Example C: Preparation of pharmaceutical preparation

The following ingredients were finely powdered. They were thoroughly stirred to prepare an intimate mixture. The mixture was then filled into pullulan capsules or gelatin capsules in an amount of 0.2 g per capsule to prepare capsule preparations.

| | |
|---|---|
| Isomerized hop extract | |
| prepared in Example B | 20 g |
| Corn starch | 1880 g |
| Hydrogenated rapeseed oil | 100 g |

### Example D: Preparation of food and drink (preparation of isoxanthohumol-containing beverage)

Boiling water (2 L) was added to 40 g of commercially available barley for barley tea, and the mixture was boiled for 5 min to extract barley tea. Isoxanthohumol was added to the extract to concentrations of 2 mg/L, 10 mg/L, and 50 mg/L to prepare isoxanthohumol-containing barely tea beverage. The isoxanthohumol used was one prepared in "Purification from hop extract (2)" in Example A.

Likewise, 2 L of boiling water was added to 50 g of commercially available green tea leaves, and the mixture was boiled for 5 min to extract green tea. Isoxanthohumol was added to the green tea to a concentration of 10 mg/L to prepare an isoxanthohumol-containing green tea beverage.

### Evaluation Test:

### Test 1: Identification of estrogen active ingredient in beer

### 1-a) Method for measuring estrogen activity

The estrogen activity of the test compound was measured using as an index cell growth of estrogen-responsive human breast cancer-derived MCF-7 cells. The cell line used is one that expressed an estrogen receptor and enhanced cell growth in the presence of an estrogen-like substance. Therefore, the estrogen activity was measured by measuring the cell growth of the cell line with Cell Counting Kit (manufactured by DOJINDO LABORATORIES).

The measurement of cell growth of MCF-7 cells with the Cell Counting Kit was carried out by measuring the absorbance at 450 nm of formazane produced from a WST-1 reagent (2-(4,5-dimethylthiazol-2-yl)-2,5-diphenyltetrazolium, monosodium salt) incorporated in cells with a microplate reader (SOFTmax (manufactured by Molecular Devices Corporation)).

More specifically, MCF-7 cells were precultured for 3 days in a DMEM medium containing 10% of fetal calf serum (manufactured by Gibco), which had been treated using Dextran-Coated Charcoal (manufactured by Sigma) for adsorbing and removing endogenous steroids, and further containing penicillin and streptomycin (each 10000 units, 1 mg/ml, manufactured by Gibco), 0.1 mM nonessential amino acid (manufactured by Gibco), 1 mM sodium pyruvate (manufactured by Gibco), and 1 µg/ml insulin (human recombinant 27.5 USP unit/mg: (manufactured by Sigma)). A cell suspension having an MCF-7 cell concentration of 8 x 10⁴ cells/ml was prepared and was seeded on a 96 well plate in an amount of 100 µl/well. One day after the initiation of the culture, the medium in the wells was replaced by a medium containing a test compound (0.1% (v/v)) dissolved in dimethylsulfoxide, and culture was continued. On the fourth day of the culture, the medium in the wells was again replaced by a fresh medium containing the test compound. On the seventh day of the culture, the cell growth was measured with Cell Counting Kit.

A dimethylsulfoxide solution of 17β-estradiol (manufactured by Sigma), which is the most potent endogenous estrogen, was used as a positive control substance.

### 1-b) Search for estrogen active component in beer

Beer was degassed and was applied to ENVI-18 column (10 g, 60 ml, manufactured by Supelco), and elution was successively carried out with 180 ml of a 20% aqueous methanol solution, 180 ml of a 50% aqueous methanol solution, 180 ml of a 80% aqueous methanol solution, and 180 ml of methanol.

Each fraction was measured for estrogen activity by the above method. As a result, activity was observed in each eluted fractions of 80% methanol and methanol.

The 80% methanol eluted fraction and the methanol eluted fraction were combined, and the combined fractions were evaporated under the reduced pressure to dryness, followed by column chromatography on silica gel (Kiesel gel 60, 10 g, manufactured by Merck) in which elution was carried out stepwise with n-hexane, a mixed solvent composed of ethyl acetate : n-hexane = 1 : 7, a mixed solvent composed of ethyl acetate : n-hexane = 1 : 4, and ethyl acetate.

The fractions thus obtained were measured for estrogen activity. As a result, activity was observed in the ethyl acetate : n-hexane = 1:7 fraction and the ethyl acetate : n-hexane = 1 : 4 fraction.

The fractions which had been found to be active were evaporated under the reduced pressure to dryness, followed by dissolution in methanol. The solution was fractionated by HPLC under the following conditions.
Mobile phase: liquid A (acetonitrile), liquid B (distilled water), and linear gradient elution with liquid A (40% to 100%)
Column: YMC-hydrosphere C18 10 x 250 mm (manufactured by YMC Co., Ltd.)
Guard column: YMC-hydrosphere C18 10 x 30 mm (manufactured by YMC Co., Ltd.)
Flow rate: 2.5 ml/min, injection amount 80 µl
Detector wavelength: UV 290 nm

A fraction was collected by HPLC at 2 min intervals. The fractions were measured for the estrogen activity by the above method.

The results were as follows.

As a result, a fraction collected in a period between retention time 14 min and retention time 16 min (Fr8) and a fraction collected in a period between retention time 18 min and retention time 20 min (Fr10) were found to have estrogen activity.

Mass (MS) spectrum measurement of substances contained in Fr8 was carried out with a mass spectrometer (DX 303, manufactured by Japan Electric Optical Laboratory) by EI-MS. As a result, an ion was observed at m/z 354. This was in agreement with the standard spectrum pattern of isoxanthohumol. Further, 5 mg of Fr8 was dissolved in 600 µl of heavy dimethylsulfoxide. The solution was subjected to 1 H-NMR, 13C-NMR, and NOESY-1D measurements with a nuclear magnetic resonance (NMR) spectrum analyzer (UNITY plus type-500, manufactured by Varian, Inc.). As a result, it was confirmed that the active ingredient of Fr8 was isoxanthohumol.

Fr10 was subjected to HPLC mass (MS) spectral analysis.

The analyzer comprised a triple stage-type mass analyzer (MS/MS) (Quattro Ultima (manufactured by Micromass)) connected to HPLC (HP 1100 (manufactured by Agilent)). Conditions for HPLC were as follows.
Mobile phase: liquid A (acetonitrile), liquid B (distilled water), and linear gradient elution with liquid A (55% to 90%)
Column: Capcell Pack C18 2.0 x 150 mm (manufactured by Shiseido Co., Ltd.)
Flow rate: 0.2 ml/min, injection amount 5 µl

The mass analyzer was operated under conditions of an electrospray ionization system for ionization (ESI⁻), precursor ion 338.3, product ion 218.8, cone voltage 80V, and collision energy 20 eV.

As a result of the measurement, it was confirmed that Fr10 had a high 8-prenylnaringenin content.

### 1-c) Analysis of beer for isoxanthohumol and 8-prenylnaringenin

Beer (1 ml) was extracted twice with 5 ml of diethyl ether, followed by evaporation to dryness under nitrogen. This was dissolved in a 50% aqueous methanol solution containing 40 ppb of 2,4-dihydrochalcone as an internal standard to prepare an analyte.

The analyzer comprised a triple stage-type mass analyzer (MS/MS) (Quattro Ultima (manufactured by Micromass)) connected to HPLC (HP 1100 (manufactured by Agilent)). Conditions for HPLC were as follows.
Mobile phase: liquid A (acetonitrile), liquid B (distilled water), and linear gradient elution with liquid A (55% to 90%)
Column: Capcell Pack C18 2.0 x 150 mm
Flow rate: 0.2 ml/min, injection amount 5 µl

The mass analyzer was operated under conditions of an electrospray ionization system for ionization (ESI⁻), and both the compounds were quantitatively determined in MRM mode at a time. For isoxanthohumol, determined ion and ionization conditions were precursor ion 352.8, product ion 232.8, cone voltage 80 V, and collision energy 18 eV. For 8-prenylnaringenin, determined ion and ionization conditions were precursor ion 338.3, product ion 218.8, cone voltage 80 V, and collision energy 20 eV.

As a result, the concentration of isoxanthohumol in beer used in the test was 2.1 mg/L, and the concentration of 8-prenylnaringenin was 43 µg/L.

### Test 2: Evaluation of in vitro estrogen activity

For purified isoxanthohumol and chemically synthesized 8-prenylnaringenin, the estrogen activity was measured in the same manner as in test 1, except that the concentrations of the test compound in the medium for MCF-7 cell culture were set as follows.

Isoxanthohumol was added so that the concentration of isoxanthohumol in the medium was 1.0 x 10⁻⁵, 1.0 x 10⁻⁶, 1.0 x 10⁻⁷, 1.0 x 10⁻⁸, 1.0 x 10⁻⁹, and 1.0 x 10⁻¹⁰ mol/L.

8-Prenylnaringenin was added so that the concentration of 8-prenylnaringenin in the medium was 1.0 x 10⁻⁷, 1.0 x 10⁻⁸, 1.0 x 10⁻⁹, 1.0 x 10⁻¹⁰, 1.0 x 10⁻¹¹, and 1.0 x 10⁻¹² mol/L.

17β-Estradiol as a positive control was added so that the concentration of 17β-estradiol in the medium was 6.3 x 10⁻¹¹, 1.6 x 10⁻¹¹, 3.9 x 10⁻¹², 1.0 x 10⁻¹², 1.0 x 10⁻¹³, and 1.0 x 10⁻¹⁴ mol/L.

The results were as shown in Fig. 1.

Both isoxanthohumol and 8-prenylnaringenin had estrogen activity. These activities were compared with the activity of estradiol. As a result, the activity was about 10⁻⁵ time for isoxanthohumol and was 10⁻² time for 8-prenylnaringenin:

### Test 3: Evaluation of in vivo estrogen activity

### 3-a) Evaluation in immature rats

*In vivo* estrogen activity was evaluated using young Wistar female rats.

The rats used were provided as follows. At the outset, 20-day old Wistar female rats (available from Charles River Japan, Inc.) were selected so that the difference in body weight among the animals was within ±10% of the average body weight. Thereafter, the animals were randomly grouped so that each group consisted of 6 animals. The animals were reared with free feeding of water and AIN-76A (manufactured by CLEA JAPAN INC.).

After preliminary rearing for one day, isoxanthohumol or 8-prenylnaringenin was forcibly orally administered into the stomach through a stomach tube at a dose of 1, 5, or 25 mg/kg/day (liquid amount 10 ml/kg) once a day for 3 days. For the control group, a solvent (peanut oil) was orally administered in the same manner as described, and for the positive control, 17β-estradiol was provided and was orally administered at a dose of 400 µg/kg/day in the same manner as described above. About 24 hr after the final administration, the uterine was harvested from the rats, and the weight of the uterine was measured to measure the *in vivo* estrogen activity.

The results were as shown in Fig. 2.

It was found that, unlike estradiol or 8-prenylnaringenin, isoxanthohumol did not have *in vivo* estrogen activity for immature rats or had only a very low level of activity.

### 3-b) Evaluation in ovariectomized rats

Ovaries on both sides of 9-week old female SD rats (available from Charles River Japan, Inc.) were removed, and the rats were randomly grouped so that each group consisted of 6 animals. The animals were reared with free feeding of water and AIN-76A (manufactured by CLEA JAPAN INC.). From the next day of the operation, the test compound was forcibly orally administered into the stomach through a stomach tube (liquid amount 10 ml/kg) once a day for 28 days.

Isoxanthohumol was dissolved in a 0.8% aqueous ethanol solution so that, as compared with the concentration of isoxanthohumol contained in beer, the resultant solutions had 1-fold, 5-fold and 20-fold isoxanthohumol concentrations. For the control group and sham operation group, the 0.8% aqueous ethanol solution was orally administered in the same manner as in the test compound administration group. 17β-Estradiol was provided as a positive control and was subcutaneously administered at a dose of 2 µg/kg/day.

The results were as shown in Figs. 3 and 4.

For the ovariectomized group (OVX), the weight of the uterine was significantly lower than that for the sham operation group, and, even for the isoxanthohumol administration group, significant recovery of the weight of the uterine was not observed.

### Test 4: Effect of ameliorating bone mineral density reduction in osteoporosis after removal of ovaries

Ovaries on both sides of 9-week old female SD rats were removed, and, from the next day of the ovary removal, the test compound was orally administered into the stomach through a stomach tube (liquid amount 10 ml/kg) once a day for 28 days.

For the control group and sham operation group, the 0.8% aqueous ethanol solution was orally administered in the same manner as in the test compound administration group. 17β-Estradiol was provided as a positive control and was subcutaneously administered at a dose of 4 µg/kg/day.

Beer (domestically produced pilsner beer) contained 2.1 mg/L isoxanthohumol and 43 µg/L 8-prenylnaringenin. Here this beer was once lyophilized and was then dissolved in water to remove ethanol to give a test compound which was then used in the test.

Isoxanthohumol was dissolved in a 0.8% aqueous ethanol solution so that, as compared with the concentration of isoxanthohumol contained in beer, the resultant solutions had 1-fold, 5-fold and 20-fold isoxanthohumol concentrations.

Further, in order to identify major ingredients which contribute to prophylactic activity against osteoporosis in beer, the following test liquids were prepared and used in the test:
· a liquid prepared by diluting a hop extract (Iso-Extract (manufactured by Hopsteiner)) having an iso-α acid content of 30% with a 0.8% aqueous ethanol solution and adjusting the diluted liquid to an iso-α acid concentration equal to the concentration of iso-α acid in beer (30 mg/L);
· a liquid prepared by diluting 8-prenylnaringenin with water so that the concentration of 8-prenylnaringenin was equal to the concentration of 8-prenylnaringenin in beer; and
· a liquid prepared by diluting xanthohumol (manufactured by Apin chemical), an isomer of isoxanthohumol, with water so that the concentration of xanthohumol was equal to the concentration of isoxanthohumol in beer (2.1 mg/L).

The bone mineral density was measured by dissecting the femur of rats and identifying the cancellous bone mineral density at the distal metaphysis bone end with a pQCT (Peripheral Quantitative computed tomography) bone mineral density measuring apparatus (XCT-960A, manufactured by Norland Stratec).

The results were as shown in Figs. 5 and 6.

For the ovariectomized group (OVX), the cancellous bone mineral density in the femur was reduced. The bone mineral density reduction, however, was significantly ameliorated by the administration of beer and isoxanthohumol. This amelioration was not observed in 8-prenylnaringenin for which estrogen activity contribution ratio in beer is considered to be the highest. For iso-α acids containing xanthohumol and isohumulons, the bone resorption inhibitory activity was already been reported through the pit formation assay. Also for these iso-α acids, any activity as in isoxanthohumol was not observed.

As is also apparent from test 3, for the ovariectomized rats, isoxanthohumol did not exhibit uterine weight increasing activity at such a dose that exhibited bone mineral density reduction inhibitory activity, and even at a dose of 20 times that dose. Further, for immature rats, isoxanthohumol did not exhibit uterine weight increasing activity at such a dose that was 1250 times larger than the dose at which the bone mineral density reduction inhibitory activity was observed.

### Test 5: Effect of ameliorating rat bone mineral density reduction by isoxanthohumol-containing beverages

Ovaries on both sides of 9-week old female SD rats were removed, and, from the next day of the ovary removal, the test solution was orally administered into the stomach through a stomach tube (liquid amount 10 ml/kg) once a day for 28 days.

For the control group and sham operation group, water was orally administered in the same manner as in the test solution administration group. 17β-Estradiol dissolved in a peanut oil was used as a positive control and was subcutaneously administered at a dose of 10 µg/kg/day.

The following test solutions were provided according to Example D:
· barley tea which had been extracted in the same manner as in Example D and was free from isoxanthohumol;
· barley tea containing 2 mg/L, 10 mg/L, and 50 mg/L isoxanthohumol;
. green tea which had been extracted in the same manner as in Example D and was free from isoxanthohumol; and
· green tea having an isoxanthohumol content of 10 mg/L.

The bone mineral density was measured in the same manner as in test 4.

The results were as shown in Figs. 7 and 8.

As compared with the sham operation group, for the ovariectomized group (OVX), the cancellous bone mineral density in the femur was reduced. However, the bone mineral density of the group to which the isoxanthohumol-containing beverage was administered was significantly higher than that of the control group to which water was administered, demonstrating that the isoxanthohumol-containing beverage had the effect of ameliorating a bone mineral density reduction. On the other hand, the isoxanthohumol-free beverage did not have the effect of ameliorating the bone mineral density reduction.

Green tea is generally said to have a large amount of tea catechin which is known to inhibit the activity of osteoclasts involved in bone resorpiton action (Biochem Biophys Res Commun 292 (1), 94-101 (2002)). For the isoxanthohumol-free green tea beverage, however, any bone mineral density reduction inhibitory effect was not observed in ovariectomized rats. Also in the case of green tea, the effect of ameliorating the bone mineral density reduction was attained only by adding isoxanthohumol (Fig. 8).

## Claims

1. Use of isoxanthohumol for the manufacture of a composition for the inhibition or prevention of a bone mineral density reduction.

2. Use according to claim 1, wherein the composition comprises an isoxanthohumol-containing isomerized hop extract as an active agent.

3. Use according to claim 2, wherein said isomerized hop extract has been produced by isomerizing a xanthohumol-containing hop extract obtained by extraction with an ethanol solvent.

4. Use according to claim 3, wherein said isomerization is carried our by heating the hop extract in alkaline water under reflux.

5. Use according to any one of claims 1 to 4, wherein the composition is a pharmaceutical composition.

6. Use according to any one of claims 1 to 5 for the prevention, treatment or amelioration of osteoporosis.

7. Use according to claims 1 to 6, wherein the composition is a food or drink.

8. Use according to claim 7, wherein said food or drink is a health food, a functional food, a food for specified health use, or a food for sick persons.

9. Use according to any one of claims 7 or 8, wherein said food or drink is in the form of a beverage.

10. Use according to claim 9, wherein said food or drink is a non-hop beverage.

11. Use according to claim 10, wherein said food or drink is a tea beverage.

12. Use according to claim 10, wherein said food or drink is a milk beverage.

13. Use according to claim 7 or 8, wherein said food or drink is yoghurt.

14. Use according to any of claims 7 to 13, wherein said composition comprises isoxanthohumol in such an amount that the intake of isoxanthohumol per day per adult is 0.003 to 0.5 mg/kg-weight.

15. Use according to any of claims 7 to 13, wherein said composition comprises isoxanthohumol in such an amount that the intake of isoxanthohumol per time is 0.2 to 30 mg.

16. Use according to claim 14 or 15, wherein said food or drink is a non-grain-hop fermentation-type food and drink.

## Patentansprüche

1. Verwendung von Isoxanthohumol zur Herstellung einer Zusammensetzung zum Verhindern oder Vorbeugen einer Knochenmineraldichte-Verminderung.

2. Verwendung gemäß Anspruch 1, wobei die Zusammensetzung als Wirkstoff einen Isoxanthohumol-haltigen isomerisierten Hopfenextrakt umfaßt.

3. Verwendung gemäß Anspruch 2, wobei der isomerisierte Hopfenextrakt durch Isomerisieren eines Xanthohumolhaltigen Hopfenextrakts hergestellt wird, der durch Extraktion mit einem Ethanollösungsmittel erhalten wird.

4. Verwendung gemäß Anspruch 3, wobei die Isomerisierung durch Erwärmen des Hopfenextrakts in alkalischem Wasser unter Rückfluß durchgeführt wird.

5. Verwendung gemäß einem der Ansprüche 1 bis 4, wobei die Zusammensetzung eine pharmazeutische Zusammensetzung ist.

6. Verwendung gemäß einem der Ansprüche 1 bis 5 zur Vorbeugen, Behandeln oder Bessern von Osteoporose.

7. Verwendung gemäß einem der Ansprüche 1 bis 6, wobei die Zusammensetzung eine Eßware oder ein Getränk ist.

8. Verwendung gemäß Anspruch 7, wobei die Eßware oder das Getränk Reformkost, ein Funktionsnahrungsmittel, ein Nahrungsmittel für spezielle Gesundheitsanwendung oder ein Nahrungsmittel für erkrankte Personen ist.

9. Verwendung gemäß einem der Ansprüche 7 oder 8, wobei die Eßware oder das Getränk in Form eines Getränks vorliegt.

10. Verwendung gemäß Anspruch 9, wobei die Eßware oder das Getränk ein Nicht-Hopfengetränk ist.

11. Verwendung gemäß Anspruch 10, wobei die Eßware oder das Getränk ein Teegetränk ist.

12. Verwendung gemäß Anspruch 10, wobei die Eßware oder das Getränk ein Milchgetränk ist.

13. Verwendung gemäß Anspruch 7 oder 8, wobei die Eßware oder das Getränk Joghurt ist.

14. Verwendung gemäß einem der Ansprüche 7 bis 13, wobei die Zusammensetzung Isoxanthohumol in einer solchen Menge umfaßt, daß die Aufnahme von Isoxanthohumol pro Tag pro Erwachsenem 0,003 bis 0,5 mg/kg Körpergewicht beträgt.

15. Verwendung gemäß einem der Ansprüche 7 bis 13, wobei die Zusammensetzung Isoxanthohumol in einer solchen Menge umfaßt, daß die Einnahme von Isoxanthohumol jeweils 0,2 bis 30 mg beträgt.

16. Verwendung gemäß Anspruch 14 oder 15, wobei die Eßware oder das Getränk eine Nicht-Getreide/Hopfen-Eßware oder -Getränk vom Nichtgärungs-Typ ist.

## Revendications

1. Utilisation d'isoxanthohumol pour la fabrication d'une composition destinée à l'inhibition ou à la prévention d'une diminution de la densité minérale osseuse.

2. Utilisation selon la revendication 1, dans laquelle la composition comprend un extrait de houblon isomérisé contenant de l'isoxanthohumol en tant qu'agent actif.

3. Utilisation selon la revendication 2, dans laquelle ledit extrait de houblon isomérisé a été produit par isomérisation d'un extrait de houblon contenant du xanthohumol obtenu par extraction avec un solvant éthanolique.

4. Utilisation selon la revendication 3, dans laquelle ladite isomérisation est effectuée en chauffant l'extrait de houblon dans de l'eau alcaline avec reflux.

5. Utilisation selon l'une quelconque des revendications 1 à 4, dans laquelle la composition est une composition pharmaceutique.

6. Utilisation selon l'une quelconque des revendications 1 à 5, pour la prévention, le traitement ou l'amélioration de l'ostéoporose.

7. Utilisation selon les revendications 1 à 6, dans laquelle la composition est un aliment ou une boisson.

8. Utilisation selon la revendication 7, dans laquelle ledit aliment ou ladite boisson est un aliment santé, un aliment fonctionnel, un aliment pour une utilisation de santé précise, ou un aliment destiné à des personnes malades.

9. Utilisation selon l'une quelconque des revendications 7 ou 8, dans laquelle ledit aliment ou ladite boisson est sous la forme d'une boisson.

10. Utilisation selon la revendication 9, dans laquelle ledit aliment ou ladite boisson est une boisson non à base de houblon.

11. Utilisation selon la revendication 10, dans laquelle ledit aliment ou ladite boisson est une boisson à base de thé.

12. Utilisation selon la revendication 10, dans laquelle ledit aliment ou ladite boisson est une boisson à base de lait.

13. Utilisation selon la revendication 7 ou 8, dans laquelle ledit aliment ou ladite boisson est du yoghourt.

14. Utilisation selon l'une quelconque des revendications 7 à 13, dans laquelle ladite composition comprend de l'isoxanthohumol en une quantité telle que la prise d'isoxanthohumol par jour pour un adulte est de 0,003 à 0,5 mg/kg de poids.

15. Utilisation selon l'une quelconque des revendications 7 à 13, dans laquelle ladite composition comprend de l'isoxanthohumol en une quantité telle que la prise d'isoxanthohumol en une fois est de 0,2 à 30 mg.

16. Utilisation selon la revendication 14 ou 15, dans laquelle ledit aliment ou ladite boisson est un aliment et une boisson de type à fermentation non à base de graines de houblon.
